# EUROPÄISCHE PATENTSCHRIFT

(11) **EP 1 331 014 B1**
(45) Veröffentlichungstag und Bekanntmachung des Hinweises auf die Patenterteilung: **15.02.2012**
(21) Anmeldenummer: 02012015.0
(22) Anmeldetag: 31.05.2002
(51) Int. Cl.: A61L 9/03, A61L 9/12, A01M 1/20

(54) **Vorrichtung zur Halterung eines in einem Behälter für mittels einer Verdampfungsvorrichtung zu verdampfende flüchtige Substanzen, insbesondere Insektizide und/oder Duftstoffe, einsetzbaren Dochtes**
Device for holding a wick in a container, for evaporating fluids, particularly insecticides and/or perfumes
Dispositif pour maintenir une mèche dans un récipient, pour vaporiser des substances liquides, notamment insecticides et/ou parfums

(30) Priorität: 16.01.2002 US 50694
(43) Veröffentlichungstag der Anmeldung: 30.07.2003
(73) Patentinhaber: C.T.R. Consultoria, Técnica e Representações Lda., 21-35-301 Samora Correia (PT)
(72) Erfinder: Vieira, Pedro Queiroz, 2775-178 Parede (PT)
(74) Vertreter: Liebl, Thomas

(56) Entgegenhaltungen:
- FR-A- 2 804 662
- US-A- 4 621 768
- US-A- 5 725 152
- US-B1- 6 236 807

## Beschreibung

Die Erfindung betrifft eine Vorrichtung zur Halterung eines in einem Behälter für mittels einer Verdampfungsvorrichtung zu verdampfende flüchtige Substanzen, insbesondere Insektizide und/oder Duftstoffe, einsetzbaren Dochtes nach dem Oberbegriff des Anspruchs 1.

Behälter für mittels einer Verdampfungsvorrichtung zu verdampfende flüchtige Substanzen sind allgemein bekannt und werden regelmäßig in ein Gehäuse einer Verdampfungsvorrichtung eingesetzt, z. B. eingeschraubt oder eingeclipst. Der Behälter umfasst einen Docht, der mit einem Ende in die zu verdampfende flüchtige Substanz ragt und mit einem gegenüberliegenden Ende aus dem Behälter hervorsteht, z. B. in eine entsprechende Dochtaussparung eines Heizelements der Verdampfungsvorrichtung hineinragt. Über den Docht wird die zu verdampfende flüchtige Substanz aufgrund der Kapillarwirkung aus dem Behälter gefördert, so dass die Substanz durch die vom Heizelement, z. B. einem Keramikblock, abgestrahlte Wärme verdampft und über Lüftungsschlitze im Gehäuse der Verdampfungsvorrichtung in die Umgebung entweichen kann. Als zu verdampfende flüchtige Substanzen werden z. B. Insektizide und/oder Duftstoffe eingesetzt. Derartige Verdampfungssysteme werden regelmäßig zur Verdampfung von flüchtigen Substanzen in geschlossenen Räumen von Wohnungen oder Häusern eingesetzt.

Ein Problem bei derartigen Verdampfungssystemen ist, dass die zu verdampfenden flüchtigen Substanzen, insbesondere Insektizide, vor allem in größeren Mengen gefährlich für die Gesundheit von Menschen, insbesondere von Kindern, sein können. Dies ist z. B. dann der Fall, wenn der Docht in unerwünschter Weise aus dem die zu verdampfende flüchtige Substanz enthaltenden Behälter herausgezogen wird, da dadurch bei gefülltem Behälter eine größere Menge der zu verdampfenden flüchtigen Substanz zugänglich ist und z. B. ausgegossen werden kann.

Weiter besteht hier die Gefahr, dass nach dem Herausziehen des Dochtes aus z. B. einem leeren Behälter vom Benutzer selbst derartige flüchtige Substanzen in den Behälter nachgefüllt werden, die entflammbar sind und somit nicht zum Einsatz in Verdampfungsvorrichtungen geeignet sind. Des weiteren besteht hier die Gefahr, dass Insektizide nachgefüllt werden, die zur Verdampfung in geschlossenen Räumen, z. B. wegen ihrer hohen Toxizität, nicht verwendet werden dürfen.

Um einen derartigen Missbrauch vorzubeugen, ist aus der US 6,236,807 bereits eine Vorrichtung zur Halterung eines in einen Behälter für mittels einer Verdampfungsvorrichtung zu verdampfende flüchtige Substanzen, insbesondere Insektizide und/oder Duftstoffe, einsetzbaren Dochtes bekannt, die einen Docht-Halteeinsatz aufweist, der eine durchgehende Halteeinsatz-Dochtöffnung zum Durchstecken des Dochtes durch den Docht-Halteeinsatz aufweist, wobei der Halteeinsatz im in eine Behälteröffnung des Behälters, vorzugsweise im in eine durchgehende Behälteröffnung eines von einem Behälterbauch wegragenden Behälterhals, eingesetzten Zustand mit einer Halteeinsatz-Außenwand wenigstens bereichsweise in einer Anlageverbindung in einem Anlagebereich der Behälteröffnung, vorzugsweise in einem Anlagebereich an einer Behälterhals-Innenwand, anliegt. Ferner ist eine Docht-Fixiereinrichtung vorgesehen, mittels der der Docht im in den Behälter eingesetzten Zustand des Docht-Halteeinsatzes gegen ein Herausziehen aus dem Behälter gesichert ist.

Konkret ist hier die Docht-Fixiereinrichtung durch eine Nadel gebildet, die unterhalb des Halteeinsatzes im Behälterbauch radial durch den Docht hindurchgesteckt ist und mit den beiden gegenüberliegenden Nadelenden in Radialrichtung gesehen aus dem Docht hervorsteht. Der Halteeinsatz ist in die Behälteröffnung des Behälterhalses mit einem Presssitz eingepresst, so dass beim Versuch des Herausziehens des Dochtes aus dem Behälter die Nadel an einem unteren Ende des Haltereinsatzes zur Anlage kommt und dadurch einen erheblichen Widerstand gegen das Herausziehen des Dochtes ausübt. Der Aufbau ist hierbei z. B. so auszulegen, dass die Widerstandskraft gegen das Herausziehen des Dochts den örtlichen Vorschriften entspricht, z. B. einer US-Vorschrift, die eine Minimumwiderstandskraft von 15 Pfund empfiehlt.

Nachteilig bei einem derartigen Aufbau ist, dass die Nadel durch einen trockenen Docht hindurchgesteckt wird, der aus einem Fasermaterial aufgebaut ist. Im aufgequollenen, d. h. vollgesogenen Zustand des Dochtes ist dieser mehr oder weniger aufgeweicht, so dass die Gefahr besteht, dass sich die Nadel lockert sowie ggf. aus dem Docht lösen kann und somit die Fixierung des Dochtes im eingesetzten Zustand nicht mehr bzw. nicht mehr in der gewünschten Weise gegeben ist. Zudem wird hierdurch die Widerstandskraft gegen das Herausziehen verringert. Weiter handelt es sich hierbei um eine lediglich provisorische Lösung, die den Anforderungen für qualitativ hochwertige Behälter nicht genügt.

Aus der FR 2 804 662 A1 ist weiter bereits eine Vorrichtung zur Halterung eines in einem Behälter einsetzbaren Dochtes bekannt, die zwei Halteeinsätze umfasst, die nacheinander in den Behälterhals einer Behälteröffnung eingesetzt werden. Ein erster einzusetzender Halteeinsatz weist an seinem einen Ende eine Vielzahl von Fingern auf, die im fertigmontierten Zustand diesen ersten Halteeinsatz im Behälterhals halten sollen. Der Docht wird in einen zweiten Halteeinsatz eingeführt, bevor dieser zweite Halteeinsatz in den ersten Halteeinsatz eingesetzt wird. Der zweite Halteeinsatz weist Rippen auf, die beim Einsetzen des zweiten Halteeinsatzes in den ersten Halteeinsatz in den Docht gepresst werden. Zwar kann mit einer derartigen Vorrichtung eine innerhalb vorgegebener Grenzen zuverlässige Dochtfixierung im Behälter zur Verfügung gestellt werden, wobei jedoch der Bauteilaufwand hierfür und damit auch der Montageaufwand, insbesondere im Rahmen einer Großserienfertigung, sehr aufwändig ist.

Demgegenüber ist es Aufgabe der Erfindung, eine Vorrichtung zur Halterung eines in einem Behälter für mittels einer Verdampfungsvorrichtung zu verdampfende flüchtige Substanzen einsetzbaren Dochtes zu schaffen, mit der eine hochwertige und funktionssichere Dochtfixierung in einem Behälter mit relativ geringem Bauteil- und Montageaufwand möglich ist.

Diese Aufgabe wird gelöst mit den Merkmalen des Patentanspruches 1.

Gemäß Anspruch 1 ist vorgesehen, dass die Klemmverbindung in der Art eine Klemmkonusverbindung durch wenigstens zwei unterschiedliche Durchmesserbereiche an der Behälterhals-Innenwand ausgebildet ist.

Vorteilhaft kann somit aufgrund der erfindungsgemäßen Lösung eine hochwertige Klemmverbindung zur Dochtfixierung mit den ohnehin vorhandenen Bauteilen zur Verfügung gestellt werden, so dass die Bauteilvielfalt hier vorteilhaft reduziert werden kann. Denn durch die erfindungsgemäße Lösung wird durch das Zusammenwirken der ohnehin vorzusehenden Bauteile, nämlich Docht-Halteeinsatz und Behälterhals, eine hochwertige und funktionssichere Klemmfixierung des Dochtes ermöglicht. Dadurch können die Bauteilkosten reduziert werden. Zusätzlich dazu können aufgrund der Reduzierung der Bauteilvielfalt auch die Kosten im Rahmen einer Montage, insbesondere in Verbindung mit einer Großserienfertigung, deutlich reduziert werden.

Eine derartige hochwertige Klemmverbindung zur Ausbildung der Fixiereinrichtung kann zudem relativ einfach und mit hoher Funktionssicherheit im Bereich der Anlageverbindung hergestellt werden. Dies wird insbesondere dadurch erreicht, dass der Docht mit einem Halteeinsatz-Innenwandbereich verklemmt wird. Dadurch kann die Klemmverbindung an den ohnehin vorhandenen Bauteilen ausgebildet werden, so dass keine separaten Bauteile dafür geschaffen werden müssen und die Bauteilvielfalt vorteilhaft reduziert werden kann. Zudem kann hier eine für eine gute und funktionssichere Fixierung des Dochtes vorteilhafte, mehr flächige Verklemmung des Dochtes erreicht werden. Auch im aufgeweichten, aufgequollenen Zustand des Dochtes kann hier eine sehr gute und funktionssichere Verklemmung erzielt werden.

Die Klemmverbindung ist hier bezüglich der Klemmkraft so auszulegen, dass die gewünschten Vorgaben bzw. ggf. die entsprechenden Vorschriften bezüglich der Mindestwiderstandskraft gegen Ausreißen bzw. Herausreißen des Dochtes erfüllt sind. Selbstverständlich ist hier auch die Einpresskraft des Halteeinsatzes in die Behälteröffnung des Behälterhalses entsprechend abgestimmt so einzustellen, dass die gewüchten Vorgaben bzw. ggf. die Vorschriften bezüglich der Mindestwiderstandskraft auch bezüglich eines Herausreißens des Dochtes mitsamt dem Halteeinsatz erfüllt sind. Bevorzugt ist hier die Widerstandskraft gegen Herausziehen des Halteeinsatzes aus dem Behälterhals größer als die durch die Klemmverbindung auf den Docht aufgebrachte Klemmkraft. Grundsätzlich könnte jedoch auch die Widerstandskraft gegen Herausziehen des Halteeinsatzes aus dem Behälterhals kleiner sein als die Widerstandskraft gegen Herausziehen des Dochtes aus dem Halteeinsatz, sofern sichergestellt ist, dass die Widerstandskraft gegen Herausziehen des Halteeinsatzes die jeweiligen Vorschriften bezüglich der Widerstandskraft erfüllt.

Durch die Klemmkonusverbindung mit wenigstens zwei unterschiedlichen Durchmesserbereichen wird weiter erreicht, dass der Halteringeinsatz-Klemmbereich mit zunehmender Halteeinsatz-Einstecklänge aufgrund der unterschiedlichen Durchmesserbereiche radial in Richtung auf den durch den Halteeinsatz durchgesteckten Docht zu verformbar ist zur sicheren Verklemmung des Dochtes entlang des Halteeinsatz-Innenwandbereichs. Ein derartiger konkreter Aufbau ist relativ einfach zu realisieren, z. B. indem der Halteeinsatz aus einem Kunststoffmaterial im Spritzgussverfahren hergestellt wird, wobei die Verformung des Klemmbereichs vorzugsweise elastisch, d. h. mit einer Rückstellung erfolgt. Grundsätzlich wäre jedoch auch eine plastische Verformung möglich, da keine Wiederverwendung des Halteeinsatzes zumindest im Handhabungsbereich des Verbrauchers beabsichtigt und erwünscht ist. Alternativ könnten jedoch auch andere Materialien oder Materialkombinationen zur Herstellung des Halteeinsatzes verwendet werden, sofern diese geeignet sind, eine Verformung des Klemmbereichs, in der beschriebenen Art und. Weise sicherzustellen.

Gemäß einer besonders bevorzugten Ausführungsform weisen diejenigen Durchmesserbereiche an der Behälterhals-Innenwand, die näher zur Behälteröffnung hin liegen als die daran in Behälterhals-Längsachsenrichtung sowie in Richtung zum Behälterbauch angrenzenden Durchmesserbereiche einen größeren Durchmesser auf als die daran in Richtung zum Behälter bauch hin angrenzenden Durchmesserbereiche. Der Halteeinsatz-Klemmbereich ist so an die unterschiedlichen Klemmbereiche an der Behälterhals-Innenwand angepasst auszubilden, z. B. in Längsrichtung gesehen an der Außenwand glatt, d. h. ohne Abstufung durchgehend, dass dieser beim Übergang von einem Durchmesserbereich zum nächsten, benachbarten Durchmesserbereich radial in Richtung auf den Doch zu verformbar ist, um den Docht sicher zu verklemmen. Ein derartiger konkreter Aufbau, bei dem die unterschiedlichen Durchmesserbereiche an der Behälterhals-Innenwand ausgebildet sind, ist besonders einfach und preiswert herzustellen, insbesondere in Verbindung mit Behältern aus Glas. Weiter ist die Anpassung des Halteeinsatzes an eine derartig ausgebildete Behälterhals-Innenwand auf besonders einfache Weise möglich, insbesondere dann, wenn der Halteeinsatz aus einem Kunststoffmaterial im Spritzgussverfahren hergestellt ist. Dadurch wird eine besonders sichere und vorteilhafte Verklemmung des Dochtes und damit eine gute Sicherung gegen Herausziehen aus dem Behälter erreicht. Grundsätzlich könnten jedoch die unterschiedlichen Durchmesserbereiche auch im Außenwandbereich des Halteeinsatz-Klemmbereichs und die Behälterhals-Innenwand z. B. entsprechend angepasst glatt durchgehend ausgebildet sein. Dies ist jedoch insgesamt schwieriger und aufwendiger in der Herstellung

Mit derartigen unterschiedlichen Durchmesserbereichen könnte die Behälterhals-Innenwand grundsätzlich konisch ausgebildet werden, was jedoch herstellungstechnisch etwas aufwendiger ist. Besonders einfach werden die unterschiedlichen Durchmesserbereiche durch eine radial umlaufende Abstufung im Bereich der Behälterhals-Innenwand gebildet, so dass dort im wesentlichen ein oberer größerer Durchmesserbereich und ein unterer kleinerer Durchmesserbereich ausgebildet ist. Für eine besonders einfache Einführung des Halteeinsatzes kann dazu im Bereich der Abstufung eine Einführschräge mit Wirkung in Richtung auf den unteren kleineren Durchmesserbereich zu ausgebildet sein. Dadurch ergibt sich ein besonders hochwertiger Aufbau, mit dem die erfindungsgemäße Klemmverbindung besonders einfach realisiert werden kann und zudem eine gute Sicherung gegen ein Herausziehen des Dochtes erzielt wird.

Der Halteeinsatz kann an einem Ende eine umlaufende Halteeinsatz-Schulter aufweisen, die im eingesetzten Zustand des Halteeinsatzes im Bereich der Behälteröffnung am Behälteröffnungs-Randbereich des Behälterhalses aufliegt. Dadurch wird auf einfache Weise sichergestellt, dass der Halteeinsatz stets richtig positioniert in die Behälteröffnung des Behälterhalses einsteckbar ist.

Gemäß einer weiteren besonders bevorzugten Ausgestaltung der Erfindung ist an der Halteeinsatz-Außenwand wenigstens ein Rastelement vorgesehen, der mit einem entsprechend zugeordneten Rastgegenelement an der Behälterhals-Innenwand zusammenwirkt und eine vorzugsweise lösbare Rastverbindung für einen guten und sicheren Halt des Halteeinsatzes im Behälterhals ausbildet. Diese Rastverbindung kann auf besonders einfache Weise durch Einpressen des Halteeinsatzes in die Behälteröffnung des Behälterhalses hergestellt werden. Zusätzlich zu der Rastkraft kann hier für eine besonders gute Haltekraft auch eine Einpresskraft als Anlagekraft an der Behälter-Innenwand wirken. Bevorzugt ist das Rastelement an der Halteeinsatz-Außenwand als wenigstens ein umlaufender Rastring ausgebildet, dem eine entsprechend umlaufende Rastnut an der Behälterhals-Innenwand zugeordnet ist. Mit einem derartigen Aufbau lässt sich eine besonders sichere Einsteck- bzw. Einpressverbindung zwischen dem Halteeinsatz und dem Behälterhals ausbilden.

Der Klemmbereich am Halteeinsatz ist gemäß einer besonders bevorzugten Ausführungsform durch wenigstens zwei sich in Richtung der Halteeinsatz-Längsachse erstreckende und radial mit einem Spaltabstand voneinander beabstandete Klemmfinger gebildet. Durch den Spaltabstand zwischen den einzelnen Klemmfingern ist ausreichend Spiel für eine Verformung der Klemmfinger in Richtung auf den Docht zu gegeben, um diesen zu verklemmen. Derartige vorzugsweise elastisch rückstellbare Klemmfinger sind insbesondere in Verbindung mit einem Kunststoffmaterial im Spritzgussverfahren besonders einfach auf preiswerte Weise herzustellen.

Eine besonders gute und sichere Verklemmung des Dochtes ergibt sich dann, wenn an der Halteeinsatz-Innenwand im Bereich des Klemmbereichs wenigstens eine vorsprungsartige Erhebung vorgesehen ist, die bei einer Verformung des Klemmbereichs in Richtung auf den Docht zu dornartig in diesen eindrückbar ist. Gemäß einer besonders bevorzugten konkreten Weiterbildung kann hierzu an den Klemmfingern an der Halteeinsatz-Innenwand in Klemmfinger-Längsrichtung gesehen wenigstens eine Klemmstufe ausgebildet sein, deren Stufenkante bei einer Verklemmung des Dochtes in den Docht eingedrückt wird. Besonders bevorzugt können hierbei die Klemmstufen benachbarter Klemmfinger in Halteeinsatz-Längsrichtung gesehen versetzt zueinander ausgebildet sein, wobei bei mehreren Klemmfingern vorzugsweise jede zweite Klemmstufe in etwa auf gleicher Höhe liegt. Durch diesen Versatz wird eine besonders bevorzugte flächige Verteilung der Klemmkraft entlang eines Docht-Außenwandbereichs erreicht. Insgesamt kann mit derartigen Klemmstufen somit eine besonders vorteilhafte Fixierung in der Art von Wiederhaken in Verbindung mit einer flächigen Verklemmung erreicht werden.

Die Klemmstufen können dabei grundsätzlich auf unterschiedliche Art und Weise ausgebildet sein. Bevorzugt ist jedoch eine Ausbildung, bei der die Abstufung von innen nach außen abfallend verläuft.

Um den Docht bei noch nicht in die Behälteröffnung des Behälterhalses eingesetztem Halteeinsatz einfach und bequem durch den Halteeinsatz durchstecken zu können, kann im Bereich der Halteeinsatz-Dochtöffnung in Halteringeinsatz-Längsrichtung wenigstens eine radial umlaufende Einführschräge für den Docht ausgebildet sein. Ebenso kann an einem Halteeinsatzende eine Einführschräge für den Halteeinsatz in die Behälteröffnung des Behälterhalses ausgebildet sein. Dadurch wird auch das Einführen und Einstecken des Halteeinsatzes in die Behälteröffnung des Behälterhalses vereinfacht.

Grundsätzlich können der Halteeinsatz und damit die diesem zugeordnete Behälteröffnung einen runden, eckigen oder dergleichen Querschnitt aufweisen. Besonders bevorzugt für günstige Geometrien und einfache Herstellung der Bauteile ist jedoch ein Aufbau, bei der der Halteeinsatz und damit die Behälteröffnung im Behälterhals einen in etwa ringförmigen, vorzugsweise kreisringförmigen Querschnitt aufweisen.

Gemäß einer weiteren bevorzugten Ausgestaltung der Erfindung ist an einer Außenwand des Behälterhalses ein Gewinde ausgebildet, so dass auf dem Behälterhals ein Verschlussdeckel aufschraubbar ist. Dadurch wird eine separate Verkaufseinheit hergestellt, die beispielsweise in unterschiedlichen Arten von Verdampfungsvorrichtungen einsetzbar ist. Durch den Verschlussdeckel wird hierbei ein Schutz für den aus dem Behälter ragenden Docht erzielt.

Wie dies bereits eingangs in Verbindung mit dem bekannten Stand der Technik erläutert wurde, kann der fertig montierte Behälter mit einer Verdampfungsvorrichtung gekoppelt werden, so dass der Docht mit einem Dochtende im Bereich einer Heizeinrichtung angeordnet ist. Besonders bevorzugt ist der Behälter in ein Gehäuse der Verdampfungsvorrichtung einschraubbar, vorzugsweise mit dem an der Außenwand des Behälterhalses für den Verschlussdeckel ausgebildeten Gewinde, wobei das Dochtende im montierten Zustand in eine Dochtaussparung eines Heizblocks der Heizeinrichtung ragt.

Der Behälterbauch kann jede beliebige Geometrie aufweisen, d. h. z. B. rund, eckig, zylindrisch, pyramidisch etc. sein, wobei der Behälter vorzugsweise aus einem Glasmaterial und/oder Kunststoffmaterial hergestellt ist.

Die Erfindung wird nachfolgend anhand einer Zeichnung näher erläutert.

Es zeigen:
- Fig. 1: einen schematischen Querschnitt durch einen Verschlussdeckel eines Behälters,
- Fig. 2: eine schematische Seitenansicht eines Behälters,
- Fig. 3: eine schematische Draufsicht auf den Behälter gemäß Fig. 2,
- Fig. 4: eine schematischen, stark vergrößerten Querschnitt durch einen in eine Behälteröffnung eines Behälterhalses einpressbaren und in der Art einer Zylinderhülse ausgebildeten Halteeinsatzes,
- Fig. 5: eine schematische, stark vergrößerte Draufsicht auf den Halteeinsatz gemäß Fig. 4,
- Fig. 6: eine schematische, stark vergrößerte Unteransicht des Halteeinsatzes gemäß Fig. 4, und
- Fig. 7: eine schematische, stark vergrößerte Querschnittsdarstellung eines Teilausschnitts eines fertig montierten Behälters, bei dem eine Klemmverbindung den durch den Halteeinsatz durchgesteckten Docht mit einem Halteeinsatz-Innenwandbereich verklemmt und gegen Herausziehen sichert.

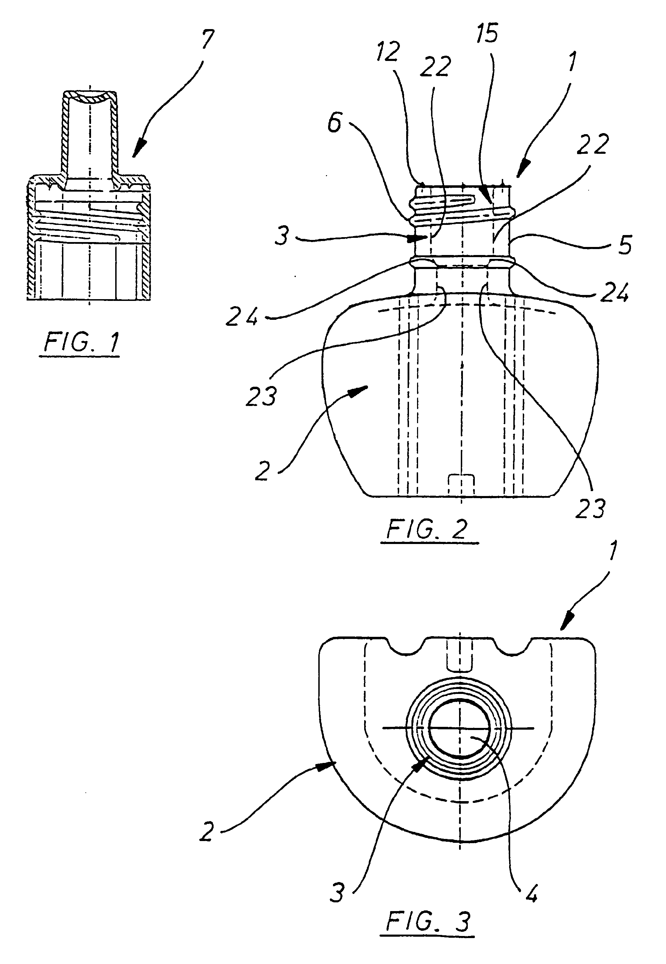

In der Fig. 2 ist schematisch eine Seitenansicht eines Behälters 1 für mittels einer hier nicht dargestellten Verdampfungsvorrichtung zu verdampfende Substanzen, wie z. B. Insektizide, gezeigt. In der Fig. 3 ist eine schematische Draufsicht auf den Behälter 1 der Fig. 2 gezeigt. Der Behälter 1 ist vorzugsweise aus einem Glasmaterial hergestellt.

Wie dies den Fig. 2 und 3 zu entnehmen ist, weist der Behälter 1 einen Behälterbauch 2 auf, in dem eine flüchtige Substanz aufgenommen werden kann. Vom Behälterbauch 2 ragt ein Behälterhals 3 weg, in dem eine Behälteröffnung 4 ausgebildet ist. Die Form des Behälterbauchs 2 ist hier lediglich beispielhaft gewählt. Grundsätzlich kann der Behälterbauch 2 mit sämtlichen gewünschten und erforderlichen Geometrien gestaltet werden.

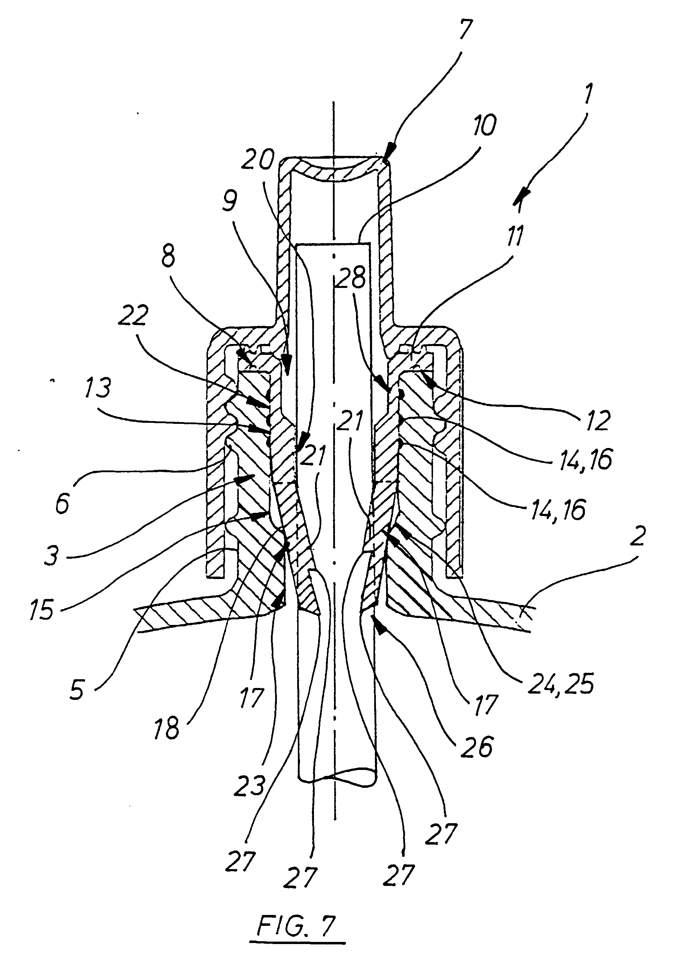

Wie dies insbesondere aus der Fig. 2 entnommen werden kann, weist der Behälterhals 3 an einer Außenwand 5 ein Gewinde 6 auf, so dass der in den Fig. 1 und 7 lediglich schematische im Querschnitt dargestellte Verschlussdeckel 7 im fertig montierten Zustand des Behälters 2 auf den Behälterhals 3 aufgeschraubt werden kann.

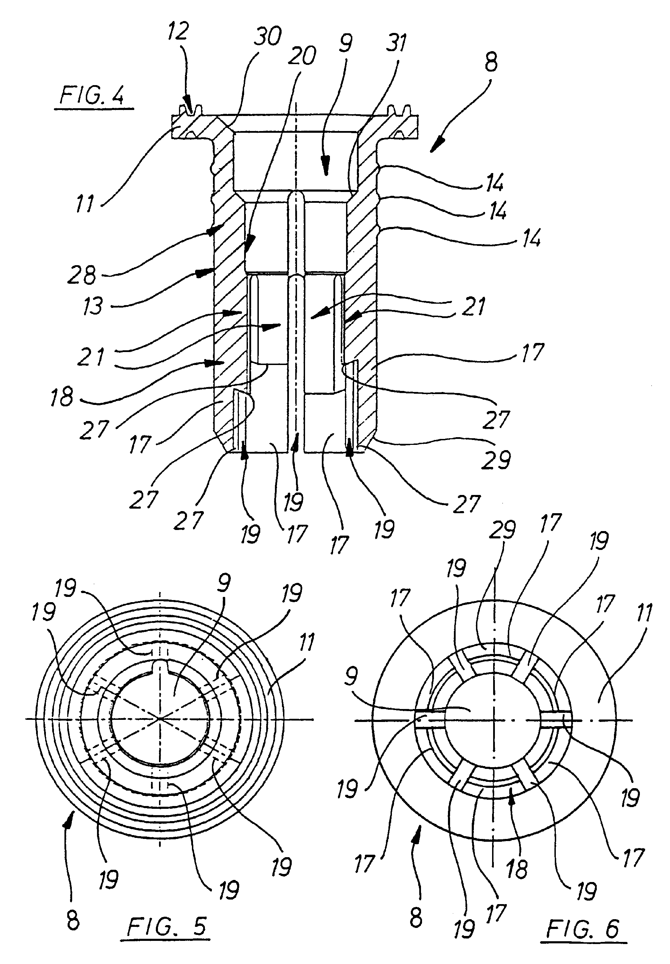

In der Fig. 4 ist eine stark vergrößerte, schematische Querschnittsdarstellung eines in die Behälteröffnung 4 des Behälterhalses 3 einpressbaren und in der Art einer Zylinderhülse ausgebildeten Docht-Halteringeinsatzes 8 als Halteeinsatz gezeigt. Dieser Halteringeinsatz 8 weist eine durchgehende Halteringeinsatz-Dochtöffnung 9 zum Durchstecken eines lediglich in der Fig. 7 gezeigten Dochtes 10 auf. In der Fig. 5 ist eine entsprechend vergrößerte schematische Draufsicht auf die entsprechend vergrößerte schematische Ansicht des Halteringeinsatzes 8 gezeigt, während in der Fig. 6 eine stark vergrößerte schematische Unteransicht gezeigt ist.

Wie dies den Fig. 4 bis 6 entnommen werden kann, weist der Halteringeinsatz 8 an einem Ende eine umlaufende Halteringeinsatz-Schulter 11 auf, die im eingesetzten Zustand des Halteringeinsatzes 8 im Bereich der Behälteröffnung 4 am Behälteröffnung-Randbereich 12 des Behälterhalses 3 aufliegt, wie dies aus der Fig. 7 ersichtlich ist. Wie dies weiter der Fig. 4 und der Fig. 7 zu entnehmen ist, sind im schulternahen Bereich an der Halteringeinsatz-Außenwand 13 drei radial umlaufende und voneinander in Längsrichtung gesehen beabstandete Rastringe 14 ausgebildet, die im in der Fig. 7 dargestellten, eingesetzten bzw. eingepressten bzw. eingerasteten Zustand des Halteringeinsatzes 8 in entsprechend zugeordnete und in der Behälteröffnung 4 des Behälterhalses 3 ausgebildete Rastnuten 16 als Rastgegenelemente eingreifen bzw. einrasten. Bevorzugt wird dabei der Halteringeinsatz 8 zumindest bereichsweise mit einem Presssitz in die Behälteröffnung 4 des Behälterhalses 3 eingepresst, wobei die durch die Rastringe 14 und die Rastnut 16 ausgebildete Rastverbindung eine zusätzliche Sicherung gegen Herausziehen des Halteringeinsatzes 8 aus der Behälteröffnung 4 ausbildet.

Wie dies insbesondere der Fig. 4 in Verbindung mit der Fig. 7 zu entnehmen ist, weist der Halteringeinsatz 8 an einem der Halteringeinsatz-Schulter 11 gegenüberliegenden Ende einen durch hier beispielhaft fünf Klemmfinger 17 gebildeten Klemmbereich 18 auf. Diese Klemmfinger 17 erstrecken sich in Richtung der Halteringeinsatz-Längsachse und weisen in Radialrichtung gesehen jeweils einen Spaltabstand 19 zum jeweils benachbarten Klemmfinger 17 auf.

Wie dies weiter insbesondere aus der Fig. 4, der Fig. 6 und der Fig. 7 ersichtlich ist, ist an jedem Klemmfinger 17 an der Halteringeinsatz-Innenwand 20 eine Klemmstufe 21 ausgebildet, wobei die Klemmstufen 21 der benachbart nebeneinander liegenden Klemmfinger 17 in Halteringeinsatz-Längsrichtung gesehen in der Höhe versetzt zueinander ausgerichtet sind, wobei jede zweite Klemmstufe 21 vorzugsweise auf einer in etwa gleichen Höhe bezogen auf die Halteringeinsatz-Längsachse liegt. Mit einer derartigen Ausbildung der Klemmstufen 21 wird erreicht, dass deren Abstufung von innen nach außen abfallend verläuft.

Den Fig. 2 und 7 kann entnommen werden, dass der Behälterhals 3 im Bereich der Behälterhals-Innenwand 15 hier beispielhaft zwei unterschiedliche Durchmesserbereiche 22, 23 aufweist, die durch eine einfache Abstufung 24 im Übergangsbereich zwischen den beiden Durchmesserbereichen 22, 23 ausgebildet ist, so dass im Bereich der Behälterhals-Innenwand 15 der Durchmesserbereich 22 einen oberen größeren Durchmesserbereich und der Durchmesserbereich 23 einen unteren kleineren Durchmesserbereich ausbilden. Wie dies insbesondere aus der Fig. 7 zu entnehmen ist, ist im Bereich der Abstufung 24 ferner eine Einführschräge 25 vorgesehen.

Die mit den unterschiedlichen Durchmesserbereichen 22, 23 ausgestaltete Behälterhals-Innenwand 15 bildet zusammen mit dem Halteringeinsatz 8 und hier insbesondere mit dem durch die Klemmfinger 17 gebildeten Klemmbereich 18 eine erfindungsgemäße Klemmverbindung 26 zur Verklemmung und Fixierung des Dochts 10 im eingesetzten Zustand aus, damit dieser gegen ein Herausziehen aus dem Behälter 1 gesichert ist. Dies wird nachfolgend ebenfalls anhand der Figuren näher erläutert:

Bevor der Halteringeinsatz 8 in die Behälteröffnung 4 des Behälterhalters 3 eingesetzt wird, befindet er sich in dem in der Fig. 4 dargestellten Zustand, bei dem der Docht 10 auf einfache Weise durch die Dochtöffnung 9 des Halteringeinsatzes 8 vorzugsweise formschlüssig durchgesteckt werden kann. Nachdem der Halteringeinsatz 8 entsprechend entlang des Dochtes 10 positioniert worden ist, wird dieser Zusammenbau aus Halteringeinsatz 8 und Docht 10 in die Behälteröffnung 10 des Behälterhalters 3 eingepresst, wobei die Klemmfinger 17 des Halteringeinsatzes 8 mit zunehmender Einstecklänge des Halteringeinsatzes 8 vom oberen Durchmesserbereich 22 ausgehend über die Einführschräge 25 in den Bereich des unteren, kleineren Durchmesserbereichs 23 überführt werden, wodurch diese, wie dies insbesondere aus der Fig. 7 ersichtlich ist, in Richtung auf den Docht 10 zu verformbar sind. Dadurch drücken sich die Stufenkanten 27 an der Innenseite der Klemmfinger 17 sowie die Klemmfinger 17 selbst wenigstens teilweise in den Docht 10 ein, wie dies in der Darstellung der Fig. 7 lediglich schematisch und prinzipiell dargestellt ist, so dass der Docht 10 gegen ein Herausziehen nach oben mit einer bestimmten Widerstandskraft gesichert ist. Die Verformung der Klemmfinger 17 des Klemmbereichs 18 wird dabei insbesondere durch den Spaltabstand 19 ermöglicht, da hierdurch entsprechender Raum für eine Verformung der Klemmfinger 17 in Richtung auf den Docht 10 zu vorhanden ist.

Die Klemmverbindung 26 ist hier somit in der Art einer Klemmkonusverbindung ausgebildet, wobei der Halteringeinsatz 8 vorzugsweise aus einem Kunststoffmaterial hergestellt ist und die Klemmfinger 17 vorzugsweise elastisch verformbar sind. Alternativ ist hier jedoch auch eine plastische Verformung möglich.

Wie dies der Fig. 7 weiter entnommen werden kann, ist der oberhalb der Klemmfinger 17 liegende obere Halteringeinsatzbereich 28, der in der Darstellung der Fig. 7 oberhalb quer der strichlierten Linie beginnt, mittels einem Presssitz in die Behälteröffnung 4 des Behälters 3 eingepresst.

Für ein vereinfachtes Einführen des Halteringeinsatzes 8 zu Beginn des Einspressvorgangs ist an einem der Schulter 11 gegenüberliegenden Ende eine Einführschräge 29 ausgebildet.

Für ein vereinfachtes Einführen des Dochtes 10 in den Halteringeinsatz 8 sind ebenfalls Einführschrägen 30, 31 vorgesehen.

## Patentansprüche

1. Vorrichtung zur Halterung eines in einen Behälter für mittels einer Verdampfungsvorrichtung zu verdampfende flüchtige Substanzen, insbesondere Insektizide und/oder Duftstoffe, einsetzbaren Dochtes,
mit einem Behälter (1), der einen Behälterhals (3) mit einer Behälteröffnung (4) aufweist,
mit einem Docht-Halteeinsatz (8), der eine durchgehende Halteeinsatz-Dochtöffnung (9) zum Durchstecken des Dochtes (10) durch den Docht-Halteeinsatz (8) aufweist und der im in die Behälteröffnung (4) des Behälterhalses (3) eingesetzten Zustand mit einer Halteeinsatz-Außenwand (13) wenigstens bereichsweise in einer Anlageverbindung an einem Anlagebereich der Behälteröffnung (4) anliegt,
mit einer Docht-Fixiereinrichtung, mittels der der Docht (10) im in den Behälter (1) eingesetzten Zustand des Docht-Halteeinsatzes (8) gegen ein Herausziehen aus dem Behälter (1) gesichert ist,
wobei der Docht (10) im nicht in die Behälteröffnung (4) eingesetzten Zustand des Halteeinsatzes (8) durch dessen Dochtöffnung (9) durchsteckbar ist,
wobei die Anlageverbindung zur Ausbildung der Fixiereinrichtung wenigstens bereichsweise als Klemmverbindung (26) ausgebildet ist dergestalt,dass die Klemmverbindung (26) im in die Behälteröffnung (4) eingesetzten Zustand des Halteeinsatzes (8) den durch den Halteeinsatz (8) durchgesteckten Docht (10) mit einem Halteeinsatz-Innenwandbereich verklemmt und gegen Herausziehen sichert, und
wobei der Halteeinsatz (8) einen verformbaren Halteeinsatz-Klemmbereich (18) aufweist dergestalt, dass der Halteeinsatz-Klemmbereich (18) mit zunehmender Halteeinsatz-Einstecklänge radial in Richtung auf den durch den Halteeinsatz (8) durchgesteckten Docht (10) zu verformbar ist zur Verklemmung des Dochtes (10) entlang des Halteeinsatz-Innenwandbereichs,
**dadurch gekennzeichnet,**
**dass** die Klemmverbindung (26) in der Art einer Klemmkonusverbindung durch wenigstens zwei unterschiedliche Durchmesserbereiche (22, 23) an der Behälterhals-Innenwand (15) ausgebildet ist.

2. Vorrichtung nach Anspruch 1, **dadurch gekennzeichnet,**
**dass** diejenigen Durchmesserbereiche (22), die näher zur Behälteröffnung (4) hin liegen als die daran in Behälterhals-Längsachsenrichtung sowie in Richtung zum Behälterbauch (2) angrenzenden Durchmesserbereiche (23) einen größeren Durchmesser aufweisen als die daran in Richtung zum Behälterbauch (2) hin angrenzenden Durchmesserbereiche (23), und
**dass** der Halteeinsatz-Klemmbereich (18) so an die unterschiedlichen Durchmesserbereiche (22, 23) an der Behälterhals-Innenwand (15) angepasst ausgebildet ist, dass dieser Halteeinsatz-Klemmbereich (18) im Verlauf des Einsteckvorgangs beim Übergang von einem Durchmesserbereich (22) zum nächsten, benachbarten Durchmesserbereich (23) radial in Richtung auf den Docht (10) zu verformbar ist zur Verklemmung desselben.

3. Vorrichtung nach Anspruch 2, **dadurch gekennzeichnet, dass** die unterschiedlichen Durchmesserbereiche (22, 23) durch eine radial umlaufende Abstufung (24) im Bereich der Behälterhals-Innenwand (15) gebildet sind, so dass dort im wesentlichen ein oberer größerer Durchmesserbereich (22) und ein unterer kleinerer Durchmesserbereich (23) ausgebildet ist.

4. Vorrichtung nach Anspruch 3, **dadurch gekennzeichnet, dass** im Bereich der Abstufung (24) eine Einführschräge (25) mit Wirkung in Richtung auf den unteren kleineren Durchmesserbereich (23) zu ausgebildet ist.

5. Vorrichtung nach einem der Ansprüche 1 bis 4, **dadurch gekennzeichnet, dass** der Halteeinsatz (8) an einem Ende eine umlaufende Halteeinsatz-Schulter (11) aufweist, die im eingesetzten Zustand des Halteeinsatzes (8) im Bereich der Behälteröffnung (4) am Behälteröffnungs-Randbereich (12) des Behälterhalses (3) aufliegt.

6. Vorrichtung nach einem der Ansprüche 1 bis 5, **dadurch gekennzeichnet, dass** an der Halteeinsatz-Außenwand (13) wenigstens ein Rastelement, (14) vorgesehen ist, der mit einem entsprechend zugeordneten Rastgegenelement (16) an der Behälterhals-Innenwand (15) zusammenwirkt und eine lösbare Rastverbindung ausbildet.

7. Vorrichtung nach einem der Ansprüche 1 bis 6, **dadurch gekennzeichnet, dass** der Klemmbereich (18) am Halteeinsatz (8) durch wenigstens zwei sich in Richtung der Halteeinsatz-Längsachse erstreckende und radial mit einem Spaltabstand (19) voneinander beabstandete Klemmfinger (17) gebildet ist.

8. Vorrichtung nach einem der Ansprüche 1 bis 8, **dadurch gekennzeichnet, dass** der Halteeinsatz-Klemmbereich (18) elastisch verformbar hergestellt ist.

9. Vorrichtung nach einem der Ansprüche 1 bis 8, **dadurch gekennzeichnet, dass** an der Halteeinsatz-Innenwand (20) im Bereich des Klemmbereichs (18) wenigstens eine vorsprungsartige Erhebung (21) vorgesehen ist, die bei einer Verformung des Klemmbereichs (18) in Richtung auf den Docht (10) zu in diesen eindrückbar ist zur Fixierung des Dochtes (10).

10. Vorrichtung nach einem der Ansprüche 7 bis 9, **dadurch gekennzeichnet, dass** an den Klemmfingern (17) an der Halteeinsatz-Innenwand (20) in Klemmfinger-Längsrichtung gesehen wenigstens eine Klemmstufe (21) ausgebildet ist, deren Stufenkante (27) bei einer Verklemmung des Dochtes (10) in den Docht (10) eingedrückt ist.

11. Vorrichtung nach Anspruch 10, **dadurch gekennzeichnet, dass** die Klemmstufen (21) benachbarter Klemmfinger (17) in Halteeinsatz-Längsrichtung gesehen versetzt zueinander ausgebildet sind.

12. Vorrichtung nach Anspruch 10 oder Anspruch 11, **dadurch gekennzeichnet, dass** die Klemmstufen (21) so ausgebildet sind, dass deren Abstufung von innen nach außen abfallend verläuft.

13. Vorrichtung nach einem der Ansprüche 1 bis 12, **dadurch gekennzeichnet, dass** im Bereich der Halteeinsatz-Dochtöffnung (9) in Halteeinsatz-Längsrichtung gesehen wenigstens eine radial umlaufende Einführschräge (30, 31) für den Docht (10) ausgebildet ist.

14. Vorrichtung nach einem der Ansprüche 1 bis 13, **dadurch gekennzeichnet, dass** an einem Halteeinsatzende eine Einführschräge (29) für den Halteeinsatz (8) in die Behälteröffnung (4) des Behälterhalses (3) ausgebildet ist.

15. Vorrichtung nach einem der Ansprüche 1 bis 14, **dadurch gekennzeichnet, dass** der Halteeinsatz (8) und damit die Behälteröffnung (4) im Behälterhals (3) einen in etwa ringförmigen Querschnitt aufweisen.

16. Vorrichtung nach einem der Ansprüche 1 bis 15, **dadurch gekennzeichnet, dass** an einer Außenwand (5) des Behälterhalses (3) ein Gewinde (6) ausgebildet ist, so dass auf den Behälterhals (3) ein Verschlussdeckel (7) aufschraubbar ist.

17. Vorrichtung nach einem der Ansprüche 1 bis 16, **dadurch gekennzeichnet, dass** der fertig montierte Behälter (1) mit einer Verdampfungsvorrichtung koppelbar ist dergestalt, dass der Docht (10) mit einem Dochtende im Bereich einer Heizeinrichtung angeordnet ist.

18. Vorrichtung nach Anspruch 17, **dadurch gekennzeichnet,**
**dass** der Behälter (1) in ein Gehäuse der Verdampfungsvorrichtung einschraubbar ist, und
**dass** das Dochtende im montierten Zustand in eine Dochtaussparung eines Heizblocks der Heizeinrichtung ragt.

19. Vorrichtung nach einem der Ansprüche 1 bis 18, **dadurch gekennzeichnet,dass** der Behälter (1) aus einem Glasmaterial und/oder einem Kunststoffmaterial hergestellt ist.

20. Vorrichtung nach einem der Ansprüche 1 bis 19, **dadurch gekennzeichnet, dass** die Widerstandskraft des Halteeinsatzes (8) gegen Herausziehen aus dem Behälterhals (3) größer ist als die Widerstandskraft des Dochtes (10) gegen Herausziehen aus dem Halteeinsatz (8).

## Claims

1. A device for holding a wick in a container, which wick can be used for volatile substances, in particular insecticides and/or aromatics, to be evaporated by an evaporation device,
having a container (1) which comprises a container neck (3) with a container opening (4),
having a wick retaining insert (8) which comprises a continuous retaining insert wick opening (9) for inserting the wick (10) through the wick retaining insert (8) and which, when inserted into the container opening (4) of the container neck (3), bears at least over portions against a contact region of the container opening (4), in a contact connection, via a retaining insert outer wall (13),
having a wick fixing means which secures the wick (10) against being pulled out from the container (1) when the wick retaining insert (8) is inserted into the container (1),
the wick (10) being insertable through the wick opening (9) in the retaining insert (8) when said retaining insert is not inserted in the container opening (4),
the contact connection for forming the fixing means being formed, at least over portions, as a clamped connection (26), in such a way that, when the retaining insert (8) is inserted into the container opening (4), the clamped connection (26) clamps the wick (10) inserted through the retaining insert (8) via a retaining insert inner wall region and secures it against being pulled out, and
the retaining insert (8) comprising a deformable retaining insert clamping region (18) in such a way that the retaining insert clamping region (18) is radially deformable, with increasing retaining insert insertion length, towards the wick (10) inserted through the retaining insert (8) to clamp the wick (10) along the retaining insert inner wall region, **characterised in that**
the clamped connection (26) is formed in the manner of a clamping cone connection by at least two different diameter regions (22, 23) at the container neck inner wall (15).

2. The device according to claim 1, **characterised in that** the diameter regions (22) located closer to the container opening (4) than the diameter regions (23) adjoining in the direction of the longitudinal axis of the container neck and towards the container belly (2) have a greater diameter than the diameter regions (23) adjoining in the direction of the container belly (2), and **in that** the retaining insert clamping region (18) is adapted to the different diameter regions (22, 23) at the container neck inner wall (15) in such a way that said retaining insert clamping region (18) is radially deformable towards the wick (10) over the course of the insertion process when transitioning from one diameter region (22) to the next adjacent diameter region (23) so as to clamp said wick.

3. The device according to claim 2, **characterised in that** the different diameter regions (22, 23) are formed by a radially circumferential gradation (24) in the region of the container neck inner wall (15) so that an upper greater diameter region (22) and a lower smaller diameter region (23) are basically formed there.

4. The device according to claim 3, **characterised in that** an insertion bevel (25) acting towards the lower smaller diameter region (23) is to be formed in the region of the gradation (24).

5. The device according to any one of claims 1 to 4, **characterised in that** the retaining insert (8) has a circumferential retaining insert shoulder (11) at one end which lies against the container opening edge region (12) of the container neck (3) when the retaining insert (8) is inserted.

6. The device according to any one of claims 1 to 5, **characterised in that** at least one snap-in element (14) is provided on the retaining insert outer wall (13) and interacts with a corresponding associated snap-in counter element (16) on the container neck inner wall (15) and forms a releasable snap-in connection.

7. The device according to any one of claims 1 to 6, **characterised in that** the clamping region (18) is formed on the retaining insert (8) by at least two clamping fingers (17) extending in the direction of the longitudinal axis of the retaining insert and spaced radially from one another by a spacing (19).

8. The device according to any one of claims 1 to 8, **characterised in that** the retaining insert clamping region (18) is resiliently deformable.

9. The device according to any one of claims 1 to 8, **characterised in that** at least one projection-like elevation (21) is provided on the retaining insert inner wall (20) in the region of the clamping region (18) and can be pressed into the wick (10) when the clamping region (18) is deformed towards said wick so as to fix the wick (10).

10. The device according to any one of claims 7 to 9, **characterised in that** at least one clamping step (21) is formed on the clamping fingers (17) on the retaining insert inner wall (20) in the longitudinal direction of the clamping fingers, the step edge (27) of which clamping step is pressed into the wick (10) when said wick (10) is clamped.

11. The device according to claim 10, **characterised in that** the clamping steps (21) of adjacent clamping fingers (17) are offset relative to each other in the longitudinal direction of the retaining insert.

12. The device according to either claim 10 or claim 11, **characterised in that** the clamping steps (21) are formed in such a way that the gradation thereof extends so as to slope from the inside out.

13. The device according to any one of claims 1 to 12, **characterised in that** a radially circumferential insertion bevel (30, 31) for the wick (10) is formed in the region of the retaining insert wick opening (9) in the longitudinal direction of the retaining insert.

14. The device according to any one of claims 1 to 13, **characterised in that** an insertion bevel (29) for insertion of the retaining insert (8) into the container opening (4) of the container neck (3) is formed at one end of the retaining insert.

15. The device according to any one of claims 1 to 14, **characterised in that** the retaining insert (8) and therefore the container opening (4) in the container neck (3) have an approximately annular cross-section.

16. The device according to any one of claims 1 to 15, **characterised in that** a thread (6) is formed on an outer wall (5) of the container neck (3) so that a closure lid (7) can be screwed onto the container neck (3).

17. The device according to any one of claims 1 to 16, **characterised in that** the finished assembled container (1) can be coupled to an evaporation device in such a way that the wick (10) is arranged with one end of the wick in the region of a heating means.

18. The device according to claim 17, **characterised in that** the container (1) can be screwed into a housing of the evaporation device, and **in that** the end of the wick protrudes into a wick recess in a heating block of the heating means when assembled.

19. A device according to any one of claims 1 to 18, **characterised in that** the container (1) is produced from a glass material and/or a plastics material.

20. The device according to any one of claims 1 to 19, **characterised in that** the force of resistance of the retaining insert (8) against being pulled out from the container neck (3) is greater than the force of resistance of the wick (10) against being pulled out from the retaining insert (8).

## Revendications

1. Dispositif pour maintenir une mèche insérable dans un récipient pour substances volatiles, notamment insecticides et/ou parfums, à vaporiser au moyen d'un dispositif de vaporisation, comprenant:
un récipient (1) qui présente un col de récipient (3) avec une ouverture de récipient (4),
un insert de maintien de mèche (8) qui présente une ouverture d'insert de maintien traversante pour la mèche (9) servant à faire passer la mèche (10) à travers l'insert de maintien de mèche (8) et qui, lorsqu'il est inséré dans l'ouverture de récipient (4) du col de récipient (3), s'appuie sur une paroi extérieure d'insert de maintien (13) au moins par secteur en liaison d'appui au niveau d'une zone d'appui de l'ouverture de récipient (4),
un dispositif de fixation de mèche, à l'aide duquel la mèche (10) est bloquée, lorsque l'insert de maintien de mèche (8) est inséré dans le récipient (1), contre une extraction du récipient (1),
dans lequel la mèche (10) peut, lorsque l'insert de maintien (8) n'est pas inséré dans l'ouverture de récipient (4), passer à travers son ouverture pour la mèche (9),
dans lequel la liaison d'appui est réalisée au moins par secteur sous forme de liaison par serrage (26) pour réaliser le dispositif de fixation de telle sorte que la liaison par serrage (26), lorsque l'insert de maintien (8) est inséré dans l'ouverture de récipient (4), serre la mèche (10) passant à travers l'insert de maintien (8) avec une zone de paroi intérieure d'insert de maintien et la bloque contre l'extraction, et
dans lequel l'insert de maintien (8) présente une zone de serrage d'insert de maintien (18) déformable de telle sorte que la zone de serrage d'insert de maintien (18) avec augmentation de la longueur d'insertion de l'insert de maintien est déformable radialement en direction de la mèche (10) passant à travers l'insert de maintien (8) pour le serrage de la mèche (10) le long de la zone de paroi intérieure d'insert de maintien,
**caractérisé en ce que**
la liaison par serrage (26) est réalisée à la manière d'une liaison par cône de serrage par au moins deux zones de diamètre (22, 23) différentes au niveau de la paroi intérieure du col de récipient (15).

2. Dispositif selon la revendication 1, **caractérisé**
**en ce que** les zones de diamètre (22) qui sont situées plus près de l'ouverture de récipient (4) que les zones de diamètre (23) qui y sont adjacentes dans le sens de l'axe longitudinal du col de récipient ainsi qu'en direction du renflement de récipient (2) présentent un diamètre supérieur à celui des zones de diamètre (23) qui y sont adjacentes en direction du renflement de récipient (2), et
**en ce que** la zone de serrage d'insert de maintien (18) est réalisée de manière adaptée aux différentes zones de diamètre (22, 23) au niveau de la paroi intérieure du col de récipient (15) de telle sorte que cette zone de serrage d'insert de maintien (18) est déformable, au cours de l'insertion lors du passage d'une zone de diamètre (22) à la prochaine zone de diamètre (23) adjacente, radialement en direction de la mèche (10) pour le serrage de cette dernière.

3. Dispositif selon la revendication 2, **caractérisé en ce que** les différentes zones de diamètre (22, 23) sont formées par un épaulement (24) radialement périphérique dans la zone de la paroi intérieure du col de récipient (15), de sorte qu'une zone de diamètre supérieure plus grande (22) et une zone de diamètre inférieure plus petite (23) y sont essentiellement réalisées.

4. Dispositif selon la revendication 3, **caractérisé en ce qu'**un chanfrein d'introduction (25) agissant en direction de la zone de diamètre inférieure plus petite (23) est réalisé dans la zone de l'épaulement (24).

5. Dispositif selon l'une quelconque des revendications 1 à 4, **caractérisé en ce que** l'insert de maintien (8) présente à une extrémité, un collet d'insert de maintien (11) périphérique qui s'appuie, lorsque l'insert de maintien (8) est inséré, dans la zone de l'ouverture de récipient (4) au niveau de la zone de bord de l'ouverture de récipient (12) du col de récipient (3).

6. Dispositif selon l'une quelconque des revendications 1 à 5, **caractérisé en ce qu'**au moins un élément d'arrêt (14), qui coopère avec un contre-élément d'arrêt (16) associé en conséquence au niveau de la paroi intérieure du col de récipient (15) et forme une liaison d'arrêt amovible, est prévu au niveau de la paroi extérieure d'insert de maintien (13).

7. Dispositif selon l'une quelconque des revendications 1 à 6, **caractérisé en ce que** la zone de serrage (18) est formée au niveau de l'insert de maintien (8) par au moins deux doigts de serrage (17) s'étendant en direction de l'axe longitudinal de l'insert de maintien et distants radialement l'un de l'autre avec une fente (19).

8. Dispositif selon l'une quelconque des revendications 1 à 8, **caractérisé en ce que** la zone de serrage d'insert de maintien (18) est fabriquée de manière élastiquement déformable.

9. Dispositif selon l'une quelconque des revendications 1 à 8, **caractérisé en ce qu'**au moins une saillie (21) en porte-à-faux, qui en cas de déformation de la zone de serrage (18) en direction de la mèche (10) peut être insérée dans celle-ci pour la fixation de la mèche (10), est prévue au niveau de la paroi intérieure d'insert de maintien (20) dans la zone de serrage (18).

10. Dispositif selon l'une quelconque des revendications 7 à 9, **caractérisé en ce qu'**au moins un gradin de serrage (21), dont l'arête de gradin (27) est enfoncée dans la mèche (10) en cas de serrage de la mèche (10), est réalisé au niveau des doigts de serrage (17) au niveau de la paroi intérieure d'insert de maintien (20) vu dans le sens longitudinal des doigts de serrage.

11. Dispositif selon la revendication 10, **caractérisé en ce que** les gradins de serrage (21) de doigts de serrage (17) adjacents sont réalisés de manière décalée l'un par rapport à l'autre vu dans le sens longitudinal de l'insert de maintien.

12. Dispositif selon la revendication 10 ou 11, **caractérisé en ce que** les gradins de serrage (21) sont réalisés de telle sorte que leur gradation s'étend de manière inclinée vers le bas de l'intérieur vers l'extérieur.

13. Dispositif selon l'une quelconque des revendications 1 à 12, **caractérisé en ce qu'**au moins un chanfrein d'introduction (30, 31) radialement périphérique pour la mèche (10) est réalisé dans la zone de l'ouverture d'insert de maintien pour la mèche (9) vu dans le sens longitudinal de l'insert de maintien.

14. Dispositif selon l'une quelconque des revendications 1 à 13, **caractérisé en ce qu'**à une extrémité d'insert de maintien, un chanfrein d'introduction (29) est réalisé pour l'insert de maintien (8) dans l'ouverture de récipient (4) du col de récipient (3).

15. Dispositif selon l'une quelconque des revendications 1 à 14, **caractérisé en ce que** l'insert de maintien (8) et ainsi l'ouverture de récipient (4) présentent dans le col de récipient (3) une section transversale à peu près annulaire.

16. Dispositif selon l'une quelconque des revendications 1 à 15, **caractérisé en ce qu'**un filetage (6) est réalisé au niveau d'une paroi extérieure (5) du col de récipient (3), de sorte qu'un couvercle de fermeture (7) peut être vissé sur le col de récipient (3).

17. Dispositif selon l'une quelconque des revendications 1 à 16, **caractérisé en ce que** le récipient (1) monté peut être couplé avec un dispositif de vaporisation de telle sorte que la mèche (10) est disposée avec une extrémité de mèche dans la zone d'un dispositif de chauffage.

18. Dispositif selon la revendication 17, **caractérisé**
**en ce que** le récipient (1) peut être vissé dans un carter du dispositif de vaporisation, et
**en ce que** l'extrémité de la mèche à l'état monté pénètre dans un évidement pour mèche d'un bloc de chauffage du dispositif de chauffage.

19. Dispositif selon l'une quelconque des revendications 1 à 18, **caractérisé en ce que** le récipient (1) est fabriqué en verre et/ou en matière plastique.

20. Dispositif selon l'une quelconque des revendications 1 à 19, **caractérisé en ce que** la résistance de l'insert de maintien (8) à l'extraction du col de récipient (3) est supérieure à la résistance de la mèche (10) à l'extraction de l'insert de maintien de mèche (8).
